# EUROPEAN PATENT SPECIFICATION

(11) **EP 2 000 167 B1**
(45) Date of publication and mention of the grant of the patent: **10.08.2016**
(21) Application number: 07740443.2
(22) Date of filing: 23.03.2007
(51) Int. Cl.: A61M 16/06

(54) **NASAL MASK FOR VENTILATION**
NASENMASKE FÜR VENTILATION
MASQUE NASAL DESTINÉ À ASSURER UNE VENTILATION

(30) Priority: 24.03.2006 JP 2006082899; 08.12.2006 JP 2006331959
(43) Date of publication of application: 10.12.2008
(73) Proprietor: Teijin Pharma Limited, Chiyoda-ku, Tokyo 100-0013 (JP)
(72) Inventor: TAKISHITA, Masahide, Hino-shi, Tokyo 191-0065 (JP); OMURA, Keiko, Hino-shi, Tokyo 191-0065 (JP); CHIN, Tongoh, Hino-shi, Tokyo 191-0065 (JP); OKAYAMA, Takamitsu, Hino-shi, Tokyo 191-0065 (JP); SHIMURA, Hideharu, Ibaraki-shi, Osaka 567-0006 (JP); FUJIMOTO, Shinya, Ibaraki-shi, Osaka 567-0006 (JP); HIKOSAKA, Toru, Ibaraki-shi, Osaka 567-0006 (JP); MATSUNAGA, Satoe, Tokyo 100-0013 (JP)
(74) Representative: Hoffmann Eitle
(86) International application number: PCT/JP2007/057005
(87) International publication number: WO 2007/111374

(56) References cited:
- WO-A1-2004/022146
- WO-A1-2005/123166
- JP-A- 2003 535 657
- JP-A- 2005 537 906
- US-A- 5 349 949

## Description

### Technical Field

The present invention relates to a nasal respiration mask used for continuous positive airway pressure (CPAP) therapy suitable for the treatment of sleep apnea syndrome, nasal intermittent positive pressure ventilation (NIPPV) therapy suitable for ventilatory insufficiency, and the like.

### Background Art

One of the most effective therapeutic methods for sleep apnea syndrome is a nasal continuous positive airway pressure (CPAP). The therapeutic method adopts respiration equipment that supplies positive-pressure gas at about 400 to 2000 Pa to the nasal cavity of a user during sleeping. Equipment that has a function to adjust pressure automatically depending on the occurrence of apnea during therapy has also been employed recently. One of the most effective therapeutic methods for ventilatory insuffciency is nasal intermittent positive pressure ventilation (NIPPV). The therapeutic method adopts respiration equipment that supplies intermittent positive-pressure gas at about 400 to 2400 Pa to the nasal cavity of a user.

When these types of equipment are used to conduct treatment, a hose to lead positive-pressure gas, a nasal respiratory mask (that is, a nasal mask cushion), a frame to retain the nasal respiratory mask at a predetermined position, and a headgear to attach the nasal respiratory mask tightly to the face are used generally to continuously supply positive pressure to the nasal cavity of a user. Various types of such a nasal respiratory mask have been disclosed for the purposes of preventing leakage of positive-pressure gas and discomfort of a user due to changes of the position of a mask frame upon body movements as well as during stationary state, and the like.

For example, Japanese Patent Laid-Open Publication No. H11-000397 proposes a nasal respiratory mask having a bellows-like elastic body and describes a bellows-like shape having several folds in its Examples. Japanese Patent Application Laid-Open Nos. 2003-535657 and 2005-537906 propose a nasal respiratory mask having a bellows-like shape with one fold to solve the above problem. These masks are effective in preventing leakage of positive-pressure gas and reducing discomfort due to pressing of a mask if they are worn appropriately during treatment.

It is required, however, to attach a nasal respiratory mask suitably to the face by adjusting the length of a strap associated with a headgear for wearing these nasal respiratory masks appropriately. This adjusting operation is conducted at the start of wearing and the adjustment is often performed in the absence of positive-pressure gas application. During treatment, however, positive pressure is applied and the bellows are inflated, thus the condition of wearing changes substantially from that in the absence of positive pressure application. That is, when the mask is worn, tightness of the headgear must be adjusted by predicting the condition of wearing under the applied positive pressure, and thus a phenomenon often occurs such that tightness of the headgear is excessive or insufficient. As a result discomfort due to excessive pressing of the mask may occur, an insufficient therapeutic effect may be obtained due to a long-term exposure to inappropriate conditions such as an increased gas leakage, or readjustment of tightness of the headgear may be required after the application of positive pressure.

Although such nasal respiratory masks are characterized in that they follow body movements during wearing, in order to secure a sufficient stroke necessary to follow the body movements during wearing, the bellows has to be made to protrude outwards more than is required to secure tight attachment at the peripherary of the nose (Japanese Patent Application Laid-Open Nos. 2003-535657 and 2005-537906) or the bellows has to be formed with several folds to position a frame away from the face (Japanese Patent Laid-open Publication No. HI 1-000397). Use of the former may narrow the field of view of a user and bring about obstacles in daily life, especially when the user must be treated by non-invasive ventilation therapy that is administered to patients with ventilatory insufficiency even during daytime.

In addition, these nasal respiratory masks are routinely washed with water for cleaning. During washing with water, a large area of the inner surface of a bellows comes into contact with each other and further tends to adhere tightly to each other due to surface tension of water, and water remains inside the bellows, which may increase the time required for drying the mask or lead unsanitary conditions. However, existing techniques represented by the above examples hardly pay attention to these points.

Further, WO 2005/123166 A1 describes a respiratory mask assembly for delivering breathable gas to a patient which includes a frame and a cushion. The cushion itself has a non-face contacting portion structured to be connected to the frame, a face-contacting portion structures to engage the patient's face and a central portion that interconnects the non-face contacting portion and the face contacting portion. The frame itself may be structured to allow a forehead support to assume multiple positions and to compensate for replacement cushions that may have a different profile, shape or size. The cushion is structured to compensate for variations in strap tension, treatment pressure and/or movement of the patient. Further a headgear may be provided with a strap assembly in which one or more straps is provided with selectively adjustable elasticity or extensibility that may automatically change in dependence of treatment pressure and/or treatment type. In this invention, changes in the wearing conditions between a state when a respiratory mask is applied to the patient's face and a state in which a positive pressure is applied may be accommodated by the provision of special headgear strap assemblies. Further, an elastic connecting part of the cushion can have various shapes and geometries.

WO 2004/022146 A1 encloses yet another respiratory mask assembly in which a cushion has a non-face contacting portion structured to be connected to a frame and a face-contacting portion structured to engage the patient's face and an intermediate portion that connects both. The intermediate portion includes a gusset portion that applies a first component of force to the patient's face through the face-contacting portion. Further, a spring structure is coupled with the face-contacting portion of the cushion, which applies a second component of force. These two components determine a contact force of the cushion applied to the patient's face. Moreover, the intermediate portion may also include an elastic cuff portion. The intermediate portion can have different shapes, but seems mainly V-shaped.

### Disclosure of the Invention

In light of various problems of the existing techniques described above, the problems to be solved by the present invention are as described below.

That is, it is an object of the present invention to provide a nasal respiratory mask that solves at least one of the following problems:
(a) to allow the mask to be worn at an appropriate position very easily without applying positive pressure at the start of wearing the mask;
(b) to reduce limitation of the field of view during wearing as far as possible;
(c) to prevent prolongation of the time required for drying or occurrence of unsanitary conditions after routine cleaning by washing with water; and
(d) to reduce the weight as far as possible while retaining the function as a nasal respiratory mask.

The problems are solved by the present invention described below.

That is, the present invention is a nasal respiratory mask according to claim 1 (that is, a nasal mask cushion) contacting with the face of a user for supplying respiratory positive-pressure gas to the nose of the user.

"Elastically connecting" used here refers to connection that is made by a shape or material that keeps an intended certain shape, that is, a shape in the conditions in which the facial side of the elastic connecting part is kept away from the frame-fitting part side thereof for a required distance in the absence of an external force and deforms more largely than other structural parts when an external force is applied. That is, the part attached tightly to the face of a nasal respiratory mask is kept away from the flame-fitting part thereof for a predetermined distance, that is, the whole length of the nasal respiratory mask is retained at a designed value in the absence of an external force. This point makes the mask of the present invention clearly different from existing flexible nasal respiratory masks that shrink in the absence of an external force.

In contrast, the nasal respiratory mask of the present invention has a structure with which the elastic connecting part deforms preferentially among the respective parts of the nasal respiratory mask, contributing to the maintenance of tight attachment of the mask to the face when an external force is applied. The external force includes a component perpendicular to the face, a component parallel to the face, and the composite component thereof

"Required distance" used here refers to a distance that can exhibit the effects of the invention of the present application, such as easiness of appropriate wearing at the start of wearing and easiness of washing with water as described above.

### Brief Description of the Drawings

Figure 1 is a drawing that explains the constitution of a conventional nasal respiratory mask 1.
Figure 2 is a drawing that explains the state in which a user wears a conventional nasal respiratory mask 1 and moves the body.
Figure 3 is a drawing that explains the state when wearing a conventional nasal respiratory mask 1 is started.
Figure 4 is a drawing that explains a suitable example of the nasal respiratory mask 2 of the present invention.
Figure 5 is a drawing that explains size reduction that is a feature of the nasal respiratory mask 2 of the present invention.
Figure 6 is a drawing that explains a suitable example of the elastic connecting part that is a feature of the nasal respiratory mask 2 of the present invention.
Figure 7 is a drawing that explains water draining that is a feature of the nasal respiratory mask 2 of the present invention.
Figure 8 is a partial cross-sectional view showing the elastic connecting part having an even thickness of the nasal respiratory mask of the present invention.
Figure 9 is a partial cross-sectional view of the nasal respiratory mask of the present invention, in which the inner side of the base part of the elastic connecting part at the part attached tightly to the face is reinforced by a rib.
Figure 10 is a partial cross-sectional view of the nasal respiratory mask of the present invention, in which the inner side of the base part of elastic connecting part at the part attached tightly to the face is reinforced by a rib.
Figure 11 is a partial cross-sectional view of the nasal respiratory mask of the present invention, in which the facial side half of the elastic connecting part is thicker than the other part.
Figure 12 is a partial cross-sectional view of the nasal respiratory mask of the present invention, in which the facial side half of the elastic connecting part is thicker than the other part.
Figure 13 is a graph in which deformation resistance under the applied respiratory positive pressure is compared between the nasal respiratory masks of the present invention shown in Figures 8 and 12.

### Explanation of Symbols

1. Conventional nasal respiratory mask
1a. Bellows-like elastic body
1b. Part attached tightly to the face
1c. Frame-fitting part
2. Respiratory nasal mask of the present invention
2a. Elastic connecting part
2b. Part attached tightly to the face
2c. Frame-fitting part
2d. Connecting part between 2a and 2b (Boundary part P)
2e. Connecting part between 2a and 2c (Boundary part Q)
2f. First arc
2g. Second arc
2h. Third arc
3. Frame
4. Headgear
4a. Headgear strap
5. Hose
6. Respiratory equipment generating positive-pressure gas
20. Water
L. Distance between 1b and 1c
La. Maximum size of L
Lb. Minimum size of L
D. Diameter of a circle when the elastic part has a circular cross-section.

### Best Mode for Carrying Out the Invention

The present invention is a nasal respiratory mask according to claim 1 contacting with the face of a user for supplying respiratory positive-pressure gas to the nose of the user (also referred simply to " positive pressure" hereinafter).

As described above, the elastic connecting part deforms preferentially to the part attached tightly to the face and the frame-fitting part when an external force is applied in the nasal respiratory mask of the present invention. Means for realizing this includes making elastic connecting in an easily deformable shape, for example, a shape having a form of the letter C as described below, using a material having low hardness only at the elastic connecting part, and the like. Further, it is preferable to combine with the adjustment of the thickness of the elastic connecting part.

The part attached tightly to the face and the frame-fitting part are required to be less deformable than the elastic connecting part, but preferably have a certain degree of elasticity.

In the nasal respiratory mask of the present invention, the cross-section of the elastic connecting part at a plane perpendicular to the face is preferably in a C-shaped arc. The convex part of the C shape is in the outward direction of the mask. Such a nasal mask can be made not larger than is necessary and reduce the distance between the face and the frame. At the same time, a stroke required for following body movements can be sufficiently secured.

In the nasal respiratory mask of the present invention, it is preferable that the cross-section of the elastic connecting part comprises at least a first arc that continues from the part attached tightly to the face, a second arc that continues from the frame-fitting part, and a third arc that directly or indirectly connects the first arc with the second arc, and the radius of the third arc is larger than the radius of the first arc and the radius of the second arc. "To connect indirectly" used here refers to the condition in which the first arc continues to the second arc via a straight-line part.

Such a nasal respiratory mask can minimize hindrance of the field of view by reducing an excessive outward protrusion of a part corresponding to the bellows part and thus further decrease discomfort during wearing.

In addition, in the nasal respiratory mask of the present invention, when a boundary part P between the elastic connecting part and the part attached tightly to the face is brought into contact with a boundary part Q between the elastic connecting part and the frame-fitting part by applying an external force, both the boundary parts P and Q preferably take a form having an acute angle. The nasal respiratory mask can. prevent sticking due to water remaining after washing and suppress prolongation of the time required for drying and occurrence of unsanitary conditions as far as possible.

The weight of the nasal respiratory mask of the present invention is preferably reduced as far as its function can be maintained. As a general technique for weight reduction, reduction of thickness of the respective parts can be considered. However, when thickness is excessively reduced, deformation tends to occur when positive pressure is applied, leading to a problem that positive-pressure gas leaks from the part attached tightly to the face.

It has been shown that the technique to reinforce the inner side of the base part of the elastic connecting part at the part attached tightly to the face by a rib is effective as one of the preferred embodiments of the present invention. That is, parts that are limited in terms of the total weight can be effectively positioned by increasing thickness only at the inner side of the base part of the elastic connecting part at the part attached tightly to the face in a form of a rib. It is desirable to form the rib part integrally in order to eliminate troubles in assembling the parts, but members provided separately may be, for example, connected by assembly or joined by adhesion.

Other means to reduce the weight of the nasal respiratory mask and to maintain resistance against deformation upon applied positive pressure at the same time include a nasal respiratory mask in which the facial side part of the elastic connecting part is made relatively thicker than the frame side part thereof. Typically, it is a nasal respiratory mask in which the facial side half is made thicker than the frame side half.

When the first arc of the elastic connecting part continues to the second arc via the third arc, it is thus preferable that the facial side part of the third arc, especially, the facial side half of the third arc is made relatively thicker than the other part of the third arc. Such a configuration also enables compatibility between reducing the weight of the nasal respiratory mask and maintaining resistance against deformation.

The present inventors obtained these findings by preparing a plurality of masks that differed in the structure of the elastic connecting part, especially the part of the third arc and the inner side of the base part of the elastic connecting part at the part attached tightly to the face and comparing these masks with each other. It is surprising that deformation resistance of the part attached tightly to the face is improved by changing the thickness of the elastic connecting part located away from the part attached tightly to the face or of the side opposite to the facial side of the part attached tightly to the face where leakage occurs, and it is difficult to explain this logically even after the above results were obtained.

As the specific thickness of the thicker part of the elastic connecting part, 0.5 to 3 mm, preferably 1.5 mm may be mentioned, and as that of the thinner part, 0.2 to 1.5 mm, preferably 0.5 mm may be mentioned. The person skilled in the art may, however, determine the most appropriate thickness for given individual conditions by performing appropriate trials by referring to these values.

The technique to reinforce the inner side of the base part of the elastic connecting part at the part attached tightly to the face by a rib and the technique to make the thickness of the facial side part of the elastic connecting part relatively larger than that of the other part of the elastic connecting part can be combined, and a nasal respiratory mask employing both techniques together is particularly preferable.

Materials of the nasal respiratory mask of the present invention are not limited as long as they are flexible and can attain the objectives described so far. However, the mask in which all of the part attached tightly to the face, the elastic connecting part and the frame-fitting part are preferably made of rubber, especially silicone rubber, and particularly liquid silicone rubber. The hardness of silicone rubber is preferably about 20 to 80 ShoreA, further preferably 30 to 50 ShoreA to exhibit effectively the capability of the part attached tightly to the face, and the like, to follow body movements. The nasal respiratory mask of the present invention is preferably formed integrally, although the part attached tightly to the face, the elastic connecting part and the frame-attaching part may be separately formed and then assembled.

The nasal respiratory mask of the present invention described above has at least one of the actions/effects described below.

That is, a change in the shape of a mask between the conditions of the presence and absence of applied positive pressure is small and the total length (length of a mask in the direction perpendicular to the face during wearing) of a nasal respiratory mask in the absence of applied positive pressure is maintained at the total length equivalent to that in the presence of applied positive pressure, so that adjustment of a headgear strap can be easily conducted to obtain a proper condition of wearing. For example, when the cross-section formed by the plane perpendicular to the face of the elastic connecting part is in a C shape directing outwards to the outer side of the mask, the mask is maintained in an inflated C shape even without applied positive pressure.

When positive pressure is applied to the nasal respiratory mask during treatment, a force pressing the part attached tightly to the face toward the face is added to the part attached tightly to the face and the elastic connecting part of the mask to further improve tight attachment.

In addition, a stroke required to follow body movements can be sufficiently secured despite a small size.

The mask also has effects of suppressing prolongation of the time required for drying and occurrence of unsanitary conditions as far as possible.

Further, when the shape of the elastic connecting part of the nasal respiratory mask of the present invention is optimized, the resistance against deformation can be maintained and leakage of positive-pressure gas from the part attached tightly to the face can be minimized while the weight of a whole nasal respiratory mask is reduced.

### Examples

The specific example of the present invention will be described in detail referring to the Drawings, but the present invention is not limited by the examples.

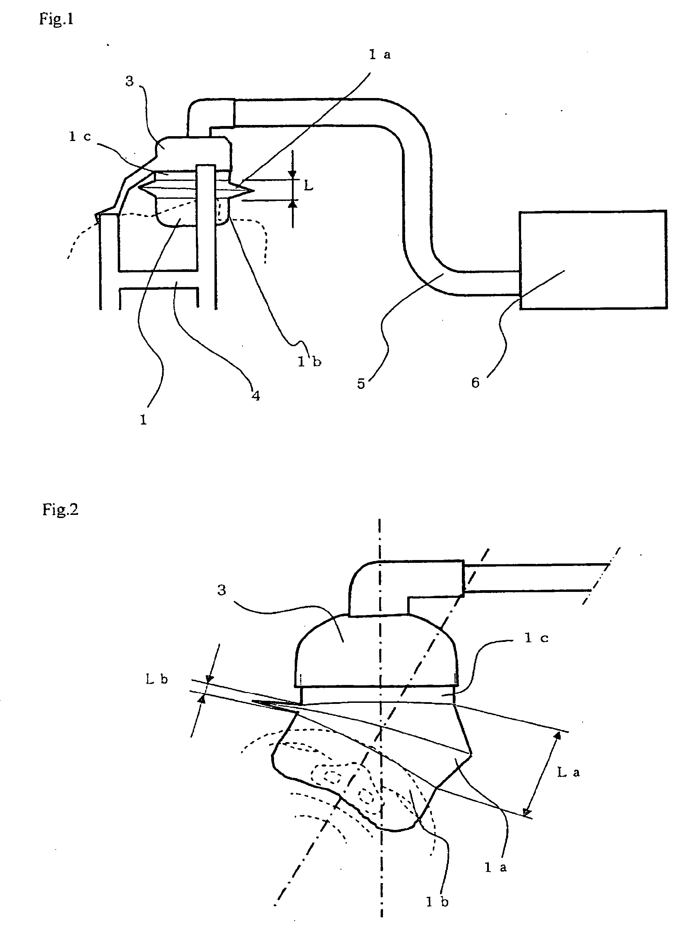

Figure 1 shows a configuration of a conventional nasal respiratory mask during use.

In Figure 1, a nasal respiratory mask 1 made of a flexible material such as silicone rubber is kept at an appropriate position by a frame 3 to fit the mask and a headgear 4 to fix the frame 3 by relying on the shape of the head. Also, the mask is used by connecting respiratory equipment 6 that generates positive-pressure gas and a hose 5 that leads the positive-pressure gas generated by the respiratory equipment 6 to the frame. The nasal respiratory mask 1 is provided with a bellows-like elastic body 1 a to keep tight attachment to the face even when the user moves the body, and when positive pressure is applied, the part attached tightly to the face 1b is kept away from the frame-fitting part 1c for a distance L.

In the above configuration, when the user moves the body, the bellows-like elastic body 1a functions as designed to deform into the state as shown in Figure 2. At the time, the distance between the part attached tightly to the face 1b and the frame-fitting part 1c varies from the maximum size La to the minimum size Lb depending on the direction of body movements. When the distance between the part attached tightly to the face 1b and the part attached tightly to the frame 1c is the minimum size Lb, the inner surface of the part attached tightly to the face is actually in contact with the inner surface of the frame-fitting part 1c, although the part attached tightly to the face 1b and the frame-fitting part 1c of the bellows-like elastic body are illustrated apart from each other for the purpose of explanation in the Drawings.

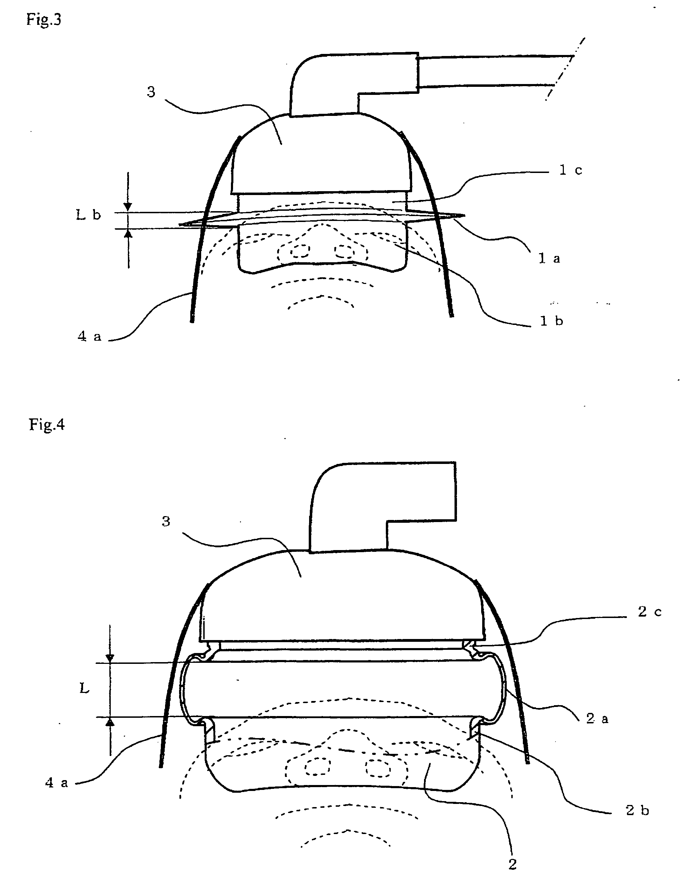

However, since positive pressure is not applied at the start of wearing, as shown in Figure 3, the bellows-like elastic body 1a is not inflated and the distance between the part attached tightly to the face 1b and the frame-fitting part 1c is equal to the minimum size Lb, and a headgear strap 4a is adjusted in this condition to adjust the degree of tight attachment of the nasal mask 1 to the face. That is, a user adjusts the headgear strap 4a while imagining an inflated condition of the bellows-like elastic body 1a after application of positive pressure, and as a result, adjustment may not sometimes be made properly. Accordingly, the headgear strap 4a is readjusted after application of positive pressure. Since the mask is not properly worn, adjustment must be made under uncomfortable conditions due to excessive pressuring of the mask or due to exposure of the eyes to positive gas leak due to insufficient tight attachment to the face. In addition, since the distance L between the part attached tightly to the face 1b and the frame-fitting part 1c is adjusted to a value without any standards, that is the middle of the maximum size La and the minimum size Lb, it is not easy for a user to judge whether or not the distance is appropriate.

On the contrary, the characteristic feature of the nasal respiratory mask of the present invention is that, as shown in Figure 4, desired capability to follow body movements is not impaired while the part attached tightly to the face 2b is kept away from the frame-fitting part 2c for the distance L, even when positive pressure is not applied.

The details of the elastic connecting part 2a that is a characteristic part of the nasal mask of the present invention 2 are explained referring to Figure 4. As shown in Figure 4, the elastic connecting part 2a has preferably a cross-section of a single arc in a C shape or multiple continuous arcs, which makes easier to separate the part attached tightly to the face 2b and the frame-fitting part 2c at the distance L in the absence of positive pressure than the conventional bellows-like elastic body 1a having an even thickness and a linear "<" shaped cross section. The distance L is preferably 5 mm to 20 mm, and the maximum effect can be obtained when the distance L is 12 mm to 20 mm.

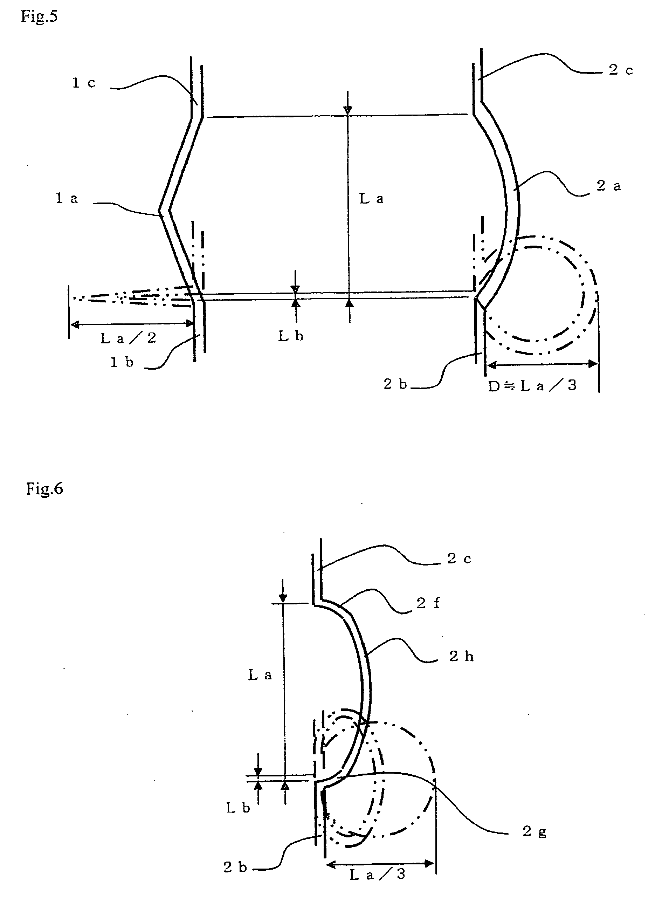

Figure 5 illustrates size reduction of a nasal mask that is one of the characteristics of the present invention. In the conventional nasal mask 1, in order to obtain the maximum distance La between the part attached tightly to the face 1b and the frame-fitting part 1c, the bellows-like elastic body 1a spreads outwards and protrudes outwards for about La/2 (see dashed double-dotted line) when the part attached tightly to the face 1 b comes into contact with the frame-fitting part 1 c. In the nasal respiratory mask of the present invention 2, however, since the elastic connecting part 2a is in an arc shape, a closed circular cross section with a diameter D is obtained when the part attached tightly to the face 2b comes into contact with the frame-fitting part 2c in order to obtain the maximum distance La. That is, the outward spread is La = πD and D is about La/3.

Further, as shown in Figure 6, an elasticity rate may be changed by composing the arc forming the cross section of the elastic connecting part 2a with a first arc 2f, a second arc 2g and a third arc 2h. Especially, when the radius of the third arc 2 h is made larger than the radius of the first arc 2f and the radius of the second arc 2g, the outward spread when the part attached tightly to the face 2b comes into contact with the frame-fitting part 2c can be made smaller than La/3 for the arc composed of a single arc. In this case, it is clear that a similar effect can be obtained when the first arc 2f is connected with the third arc 2h and the second arc 2g is connected with the third arc 2h by a linear cross section.

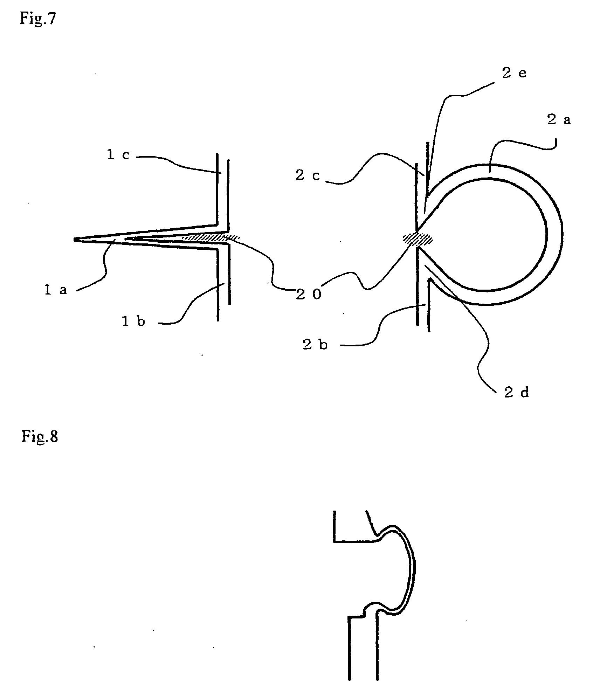

Next, water drainage, one of the characteristics of the nasal mask 2 of the present invention, will be explained referring to Figure 7. Usually, the nasal mask 2 is cleaned by washing with water using the hands of a user and attached again to the frame 3 after removal of water 20. In the conventional nasal mask 1, since a large area of the inner surface of the bellows is in contact with each other in the absence of positive pressure, tight adhesion occurred often due to surface tension of water 20 remaining after washing. In the nasal mask 2 of the present invention, water 20 hardly remains inside the elastic connecting part 2a, since the part attached tightly to the face 2b is kept away from the frame-fitting part 2c. In addition, the connecting part 2d between the part attached tightly to the face 2b and the elastic connecting part 2a, that is, a boundary part P, and the connecting part 2e between the frame-fitting part 2c and the elastic connecting part 2a, that is, a boundary part Q, are linked in an acute angle, which can significantly decrease possibilities that the elastic connecting part 2a adheres due to surface tension of water remaining after washing, or a longer time is required for drying and unsanitary condition occurs.

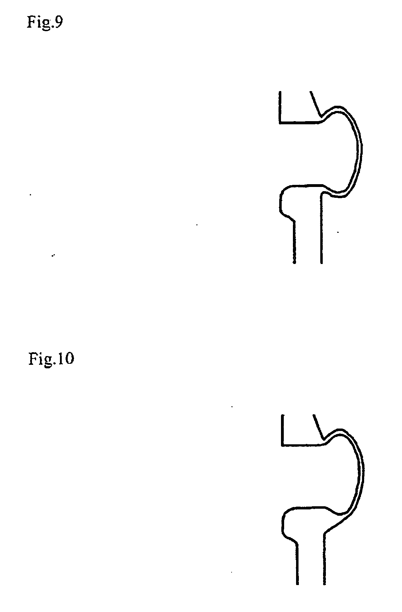

Next, the nasal respiratory mask of the present invention in which the inner side of the base part of the elastic connecting part at the part attached tightly to the face is reinforced with a rib will be explained. In the nasal respiratory mask of the present invention shown in Figure 8, the member of the facial side (that is, downside in the Drawing) continuously connecting to the elastic connecting part (the first arc continuing to the second arc via the third arc is shown here) has substantially an even thickness. On the other hand, in the nasal respiratory mask of the present invention shown in Figures 9 and 10, the inner side of the base part of the elastic connecting part at the part attached tightly to the face is reinforced with a rib (a part protruding in the left side). The base part of the elastic connecting part at the part attached tightly to the face provided with the rib may be expressed as "supporting part wall" hereinafter. In the nasal respiratory mask of the present invention shown in Figure 10, the thickness in the vicinity of the end on the facial side of the elastic connecting part is made larger.

As the shape of the rib, those having a rectangular cross section of 2 mm to 6 mm in width and 1 mm to 4 mm in length are preferable. Particularly, it is preferable that the height of the cross section of the rib including the thickness of the supporting part wall is 4 mm to 8 mm.

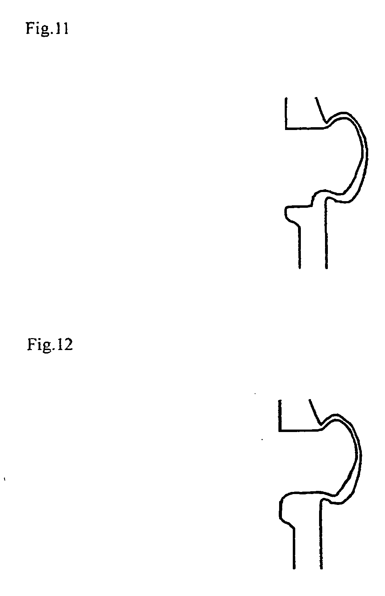

Next, the nasal respiratory mask in which the facial side half of the elastic connecting part is thicker than the other half will be explained. Although the thickness of the elastic connecting part is uniform in Figure 8, the facial side half, that is, the lower half in the Drawing, of the elastic connecting part is thicker than the other half in the nasal respiratory mask of the present invention shown in Figures 11 and 12. In the nasal respiratory mask of the present invention shown in Figures 11 and 12, a rib is provided on the inner side of the base part of the elastic connecting part at the part attached tightly to the face as described above, but the shape of the rib differs between the two.

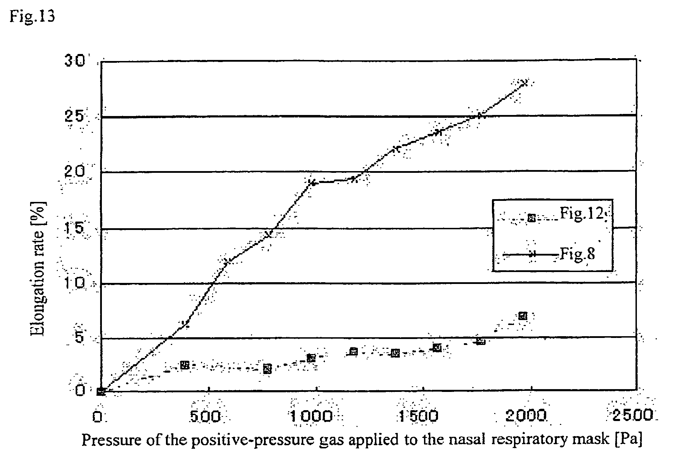

Next, the nasal respiratory mask of the present invention shown in Figure 8 is compared with the nasal respiratory mask of the present invention shown in Figure 12 in which the inner side of the base part of the elastic connecting part at the part attached tightly to the face is reinforced with a rib and the facial side half of the elastic connecting part is made thicker than the other half in terms of deformation resistance under applied positive pressure, and the results are shown in a graph in Figure 13. The axis of abscissa shows positive pressure applied to the nasal respiratory mask and the axis of the ordinate shows elongation rate. "Elongation rate" (the unit is in %) used here is a value calculated by the equation (B/A - 1) x 100, where "A" is a horizontal width at a predetermined position of a nasal respiratory mask, a target of measurement, in the absence of applied positive pressure and "B" is a horizontal width in the presence of positive pressure. "horizontal width at a predetermined position" used here is a length of a segment of a straight line divided by the other two sides of a triangle, wherein the straight line is in the same plane as an approximate isosceles triangle formed by the boundary part P between the elastic connecting part and the part attached tightly to the face of a nasal respiratory mask, is parallel to the side of the triangle corresponding to the upper part of the lip, and is 30 mm away from the tip corresponding to the nasal bridge.

Here, the shapes of the nasal respiratory mask shown in Figure 8 and that shown in Figure 12 are explained. For both masks, the shape of the frame-fitting part is the same, the thickness of the face-contacting part is basically common, 4 mm, and the distance L between 2b and 2c is also common, 10 mm. However, a rib having a cross section of 5 mm in width and 2 mm in height (6 mm including the thickness of the supporting part wall) is provided on the inner side of the base part of the elastic connecting part at the face-contacting part of the later. In addition, the two masks differ in that the thickness of all of the elastic connecting part of the former and the frame-fitting part side half of the elastic connecting part of the later is 0.7 mm while the thickness of the face-contacting part side half of the elastic connecting part of the later is 1.5 mm.

According to Figure 13, it is shown that deformation resistance of a nasal respiratory mask upon application of positive pressure is remarkably increased by slightly changing the shapes of the side kept away from the face of the face-contacting part and the elastic connecting part.

### Industrial Applicability

According to the present invention, a nasal respiratory mask used in CPAP therapy, NIPPV therapy and the like is provided.

## Claims

1. A nasal respiratory mask (2) contacting with the face of a user for supplying respiratory positive-pressure gas to the nose of the user, comprising at least a part attached tightly to the face (2b) that is attached tightly to the face of the user; a frame-fitting part (2c) engaged with a frame (3) to fix the nasal mask (2) at a predetermined position; and an elastic connecting part (2a) connecting elastically the part attached tightly to the face (2b) with the frame-fitting part (2c), wherein the cross-section formed by a plane perpendicular to the face of the elastic connecting part (2a) is in a C-shaped arc, **characterized in that** the cross-section of the elastic connecting part (2a) comprises at least a first arc (2f) continuing from the part attached tightly to the face (2b), a second arc (2g) continuing from the frame-fitting part (2c), and a third arc (2h) directly or indirectly connecting the first arc (2f) with the second arc (2g); the radius of the third arc (2h) is larger than the radius of the first arc (2f) and the radius of the second arc (2g); the facial side part of the elastic connecting part (2a) is thicker than the frame side part thereof; the facial side of the elastic connecting part (2a) is kept away from the frame-fitting part side thereof for a required distance even in the absence of applied respiratory positive-pressure.

2. The nasal respiratory mask (2) according to claim 1, wherein, when a boundary part P between the elastic connecting part (2a) and the part attached tightly to the face (2b) is made contact with a boundary part Q between the elastic connecting part (2a) and the frame-fitting part (2c) by an external force, both the boundary parts P and Q take the form of an acute angle.

3. The nasal respiratory mask (2) according to claim 1 or 2, wherein the part attached tightly to the face (2b), the elastic connecting part (2a) and the frame-fitting part (2c) are formed integrally.

4. The nasal respiratory mask (2) according to any of claims 1 to 3, wherein the required distance is 5 mm or more and 20 mm or less.

5. The nasal respiratory mask (2) according to any of claims 1 to 4, wherein the inner side of the base part of the elastic connecting part (2a) at the part attached tightly to the face (2b) is reinforced by a rib.

6. The nasal respiratory mask (2) according to claim 5, wherein the rib has a rectangular cross section of 2 mm to 6 mm in width and 1 mm to 4 mm in height.

## Patentansprüche

1. Nasale Atemmaske (2), welche das Gesicht eines Benutzers kontaktiert, zum Zuführen eines Atemgases mit positivem Druck zu der Nase des Benutzers, mit zumindest einem Teil der eng mit dem Gesicht verbunden ist (2b), der eng mit dem Gesicht des Benutzers verbunden ist, einem Rahmenbefestigungsteil (2c), der mit einem Rahmen (3) in Eingriff steht, um die nasale Maske (2) an einer vorbestimmten Position zu befestigen und einem elastischen Verbindungsteil (2a), der den eng mit dem Gesicht verbundenen Teil (2b) elastisch mit dem Rahmenbefestigungsteil (2c) verbindet, wobei der Querschnitt des elastischen Verbindungsteils (2a) ausgebildet in einer Ebene senkrecht zu dem Gesicht ein C-förmiger Bogen ist, **dadurch gekennzeichnet, dass**
der Querschnitt des elastischen Verbindungsteils (2a) zumindest einen ersten Bogen (2f), der sich von dem eng mit dem Gesicht verbundenen Teil (2b) erstreckt, einen zweiten Bogen (2g), der sich von dem Rahmenbefestigungsteil (2c) erstreckt und einen dritten Bogen (2h) aufweist, der den ersten Bogen (2f) mit dem zweiten Bogen (2g) direkt oder indirekt verbindet;
der Radius des dritten Bogens (2h) größer ist als der Radius des ersten Bogens (2f) und der Radius des zweiten Bogens (2g);
der gesichtsseitige Teil des elastischen Verbindungsteils (2a) dicker ist als der rahmenseitige Teil davon; und
eine Gesichtsseite des elastischen Verbindungsteils (2a) von der Seite des Rahmenbefestigungsteils davon über einen benötigten Abstand, selbst in der Abwesenheit von aufgebrachtem positivem Atemdruck ferngehalten wird.

2. Nasale Atemmaske (2) nach Anspruch 1, wobei
wenn ein Grenzteil P zwischen dem elastischen Verbindungsteil (2a) und dem eng mit dem Gesicht verbundenen Teil (2b) mit einem Grenzteil Q zwischen dem elastischen Verbindungsteil (2a) und dem Rahmenverbindungsteil (2c) durch eine externe Kraft in Kontakt gebracht wird, beide Grenzteile P und Q die Form eines spitzen Winkels annehmen.

3. Nasale Atemmaske (2) nach Anspruch 1 oder 2, wobei
der eng mit dem Gesicht verbundene Teil (2b), der elastische Verbindungsteil (2a) und der Rahmenanbringteil (2c) einstückig ausgebildet sind.

4. Nasale Atemmaske (2) nach einem der Ansprüche 1 bis 3, wobei
der benötigte Abstand 5 mm oder mehr und 20 mm oder weniger beträgt.

5. Nasale Atemmaske (2) nach einem der Ansprüche 1 bis 4, wobei
die innere Seite des Basisteils des elastischen Verbindungsteils (2a) an dem eng mit dem Gesicht verbundenen Teil (2b) durch eine Rippe verstärkt ist.

6. Nasale Atemmaske (2) nach Anspruch 5, wobei
die Rippe einen rechteckigen Querschnitt von 2 mm bis 6 mm in der Breite und 1 mm bis 4 mm in der Höhe aufweist.

## Revendications

1. Masque respiratoire nasal (2) en contact avec le visage d'un utilisateur pour amener un gaz respiratoire à pression positive au nez de l'utilisateur, comprenant au moins une partie fixée étroitement au visage (2b) qui est fixée étroitement au visage de l'utilisateur, une partie (2c) d'ajustement à un cadre en contact avec un cadre (3) pour fixer le masque nasal (2) dans une position prédéterminée, et une partie de raccordement élastique (2a) raccordant élastiquement la partie fixée étroitement au visage (2b) à la partie (2c) d'ajustement à un cadre, la section transversale formée par un plan perpendiculaire au visage de la partie de raccordement élastique (2a) présentant la forme d'un arc en C, **caractérisé en ce que** la section transversale de la partie de raccordement élastique (2a) comprend au moins un premier arc (2f) se poursuivant depuis la partie fixée étroitement au visage (2b), un deuxième arc (2g) se poursuivant depuis la partie d'ajustement (2c) au cadre et un troisième arc (2h) raccordant directement ou indirectement le premier arc (2f) au deuxième arc (2g) ; le rayon du troisième arc (2h) est plus grand que le rayon du premier arc (2f) et que le rayon du deuxième arc (2g) ; la partie latérale faciale de la partie de raccordement élastique (2a) est plus épaisse que la partie latérale de cadre de cette dernière ; un côté facial de la partie de raccordement élastique (2a) est maintenu à distance du côté de partie latérale de cadre de cette dernière sur une distance requise y compris en l'absence d'une pression positive respiratoire appliquée.

2. Masque respiratoire nasal (2) selon la revendication 1, où, lorsqu'une partie limite P entre la partie de raccordement élastique (2a) et la partie fixée étroitement au visage (2b) est amenée en contact avec une partie limite Q entre la partie de raccordement élastique (2a) et la partie (2c) d'ajustement au cadre par une force externe, les parties limites P et Q prennent toutes les deux la forme d'un angle aigu.

3. Masque respiratoire nasal (2) selon la revendication 1 ou 2, la partie fixée étroitement au visage (2b), la partie de raccordement élastique (2a) et la partie (2c) d'ajustement au cadre étant formées d'une seule pièce.

4. Masque respiratoire nasal (2) selon l'une quelconque des revendications 1 à 3, la distance requise étant supérieure ou égale à 5 mm et inférieure ou égale à 20 mm.

5. Masque respiratoire nasal (2) selon l'une quelconque des revendications 1 à 4, le côté intérieur de la partie de base de la partie de raccordement élastique (2a) au niveau de la partie fixée étroitement au visage (2b) étant renforcé par une nervure.

6. Masque respiratoire nasal (2) selon la revendication 5, la nervure présentant une section transversale rectangulaire de 2 mm à 6 mm de largeur et de 1 mm à 4 mm de hauteur.
